# EUROPÄISCHE PATENTSCHRIFT

(11) **EP 2 846 729 B1**
(45) Veröffentlichungstag und Bekanntmachung des Hinweises auf die Patenterteilung: **20.07.2016**
(21) Anmeldenummer: 13726439.6
(22) Anmeldetag: 07.05.2013
(51) Int. Cl.: A61C 9/00

(54) **VERFAHREN ZUR VERMESSUNG EINER ZAHNSITUATION**
METHOD FOR MEASURING A DENTAL SITUATION
PROCÉDÉ DE RELEVÉ D'UNE SITUATION DENTAIRE

(30) Priorität: 07.05.2012 DE 102012207499
(43) Veröffentlichungstag der Anmeldung: 18.03.2015
(73) Patentinhaber: Sirona Dental Systems GmbH, 64625 Bensheim (DE)
(72) Erfinder: THIEL, Frank, 64372 Ober-Ramstadt (DE); SCHNEIDER, Sascha, 64367 Mühltal (DE)
(74) Vertreter: Sommer, Peter
(86) Internationale Anmeldenummer: PCT/EP2013/059426
(87) Internationale Veröffentlichungsnummer: WO 2013/167555

(56) Entgegenhaltungen:
- EP-A1- 1 820 469
- EP-A2- 0 913 130
- WO-A1-94/00074
- DE-A1-102008 006 048
- FR-A1- 2 977 469
- US-A1- 2008 038 688

## Beschreibung

### Technisches Gebiet

Die Erfindung betrifft ein Verfahren zur Vermessung einer Zahnsituation umfassend mehrere Implantate und/oder Präparationen zum Einsetzen von Zahnrestaurationen. Unter Verwendung eines ersten Messverfahrens wird zunächst ein erster Bereich der Zahnsituation erfasst. Dabei werden erste Messdaten erzeugt. Der erste Bereich umfasst mindestens zwei Implantate und/oder Präparationen.

### Stand der Technik

Aus dem Stand der Technik sind mehrere Verfahren zur Vermessung von Zahnsituationen bekannt, bei denen die relative Position von Implantaten oder Präparationen zueinander bestimmt wird.

Typischerweise erfolgt die Vermessung von Implantatpositionen und Implantatorientierungen mittels eines konventionellen Abdrucks, wobei ein Gipsmodell erstellt wird. Das Gipsmodell wird anschließend vermessen und die Positionen und Orientierungen der Implantate werden unter Verwendung der erzeugten Messdaten bestimmt. Bei diesen Verfahren wird meist die relative Lage und Orientierung der Implantate zueinander und relativ zum Restzahnbestand bestimmt.

Bei einem alternativen Verfahren kann die Zahnsituation mittels eines optischen dreidimensionalen Messverfahrens vermessen werden, um anschließend digital die Lage und Orientierung der Implantate relativ zueinander und relativ zum Restzahnbestand zu bestimmen.

Die DE 10 2004 035 091 A1 offenbart ein Verfahren zur Bestimmung der Lage und Orientierung eines dentalen Implantats sowie ein Aufsatzteil hierfür. Dabei wird auf das Implantat eine Messgeometrie aufgesetzt, die einen Rückschluss auf die Lage und Orientierung des Implantats zulässt. Anschließend erfolgt eine dreidimensionale Vermessung der Zahnsituation, wobei Messdaten erzeugt werden. In den Messdaten wird der Messkörper identifiziert und die Lage und Orientierung des Messkörpers bestimmt. Anhand der Lage und der Orientierung der Messgeometrie werden dann anschließend die Lage und Orientierung des Implantats bestimmt. Es sind verschiedene Ausführungsformen des Aufsatzteils offenbart, das beispielsweise in Form eines Sechskants ausgestaltet sein kann.

Die DE 10 2004 035 090 A1 offenbart ein Ausgleichsteil und ein Verfahren für die Vermessung von Zahnrestaurationen. Dabei wird auf ein in einem Arbeitsmodell vorgesehenes Manipulierimplantat ein Aufsatzteil aufgesetzt, das Auflageflächen zur Auflage auf das die Zahnrestauration umgebende Zahnfleisch umfasst. Das Aufsatzteil kann eine Kennung aufweisen, die eine Bestimmung der Orientierung des Aufsatzteils ermöglicht.

Die DE 10 2007 056 820 A1 offenbart einen Messkörper für ein Implantat und ein Verfahren zur Erstellung einer 3D-Messaufnahme. Der Messkörper weist eine Messgeometrie auf, die mittels einer Messkamera erfasst wird. Anhand der Messgeometrie können dann die Ausrichtung und Orientierung des Implantats bestimmt werden.

EP 0 913 130 A2 offenbart ein Verfahren zur Herstellung eines Zahnersatzteils, wobei die Form eines primären Duplikats eines mit dem Zahnersatzteil versehenden Restzahnbereichs mittels einer Formermittlungsvorrichtung vermessen wird. Das Duplikat wird mittels einer Sensoreinrichtung in mehreren Ebenen und unter mehreren Winkeln abgetastet.

Ein sekundäres Duplikat wird in mehrere Duplikatsabschnitte zertrennt wobei die einzelnen Duplikatsabschnitte einzeln vermessen werden und die dabei ermittelten Daten gespeichert werden, wobei die Daten des Duplikats und die Daten der Duplikatsabschnitte zusammengeführt werden können. Darüber hinaus ist offenbart, dass die Ermittlung der Form des Duplikats mit einer geringeren Genauigkeit erfolgt als die Ermittlung der Form der Duplikatsabschnitte. EP 1 820 469 A1 offenbart eine Vorrichtung zum Abtasten eines Sägezahnmodells, wobei das Sägezahnmodell mehrere Modellteile aufweist. Das Abtasten erfolgt mittels einer optischen Abtasteinrichtung, wobei mit der Abtasteinrichtung erste Daten von einem Übersichtsscan, der sich über mehrere Modellteile erstreckt und zweite Daten von einem Einzelscan, der sich nur über ein Modellteil erstreckt, gewonnen werden können, wobei die ersten und die zweiten Daten des Modells mit einer unterschiedlichen Auflösung wiedergegeben werden. Anschließend werden die ersten Daten und die zweiten Daten unter Verwendung von 3D-Matchen zusammengefügt. WO 94/00074 offenbart eine Vorrichtung zur Korrelation von dreidimensionalen Daten von menschlichen Organen insbesondere zur Anwendung in der Zahnmedizin.

Ein Nachteil der genannten Verfahren besteht darin, dass die Qualität der Aufnahmen zur Bestimmung der Lage und Orientierung der Implantate relativ zueinander oft nicht ausreichend ist. Zugleich ist eine Aufnahme mit einer höheren Auflösung wegen einer damit verbundenen längeren Aufnahmezeit oft nicht möglich.

Die Aufgabe der folgenden Erfindung besteht also darin, ein Verfahren zur Vermessung einer Zahnsituation bereitzustellen, das eine genaue Bestimmung der Lage und Orientierung von Implantaten relativ zueinander ermöglicht.

### Darstellung der Erfindung

Die Erfindung betrifft ein Verfahren zur Vermessung einer Zahnsituation umfassend mehrere Implantate und/oder Präparationen zum Einsetzen von Zahnrestaurationen. Unter Verwendung eines ersten Messverfahrens wird zunächst ein erster Bereich der Zahnsituation erfasst. Dabei werden erste Messdaten erzeugt. Der erste Bereich umfasst mindestens zwei Implantate und/oder Präparationen. Anschließend werden Objektbereiche um die Implantate und/oder die Präparationen festgelegt, wobei unter Verwendung eines zweiten Messverfahrens die festgelegten Objektbereiche erfasst werden. Dabei werden zweite Messdaten erzeugt, wobei das zweite Messverfahren präziser ist als das erste Messverfahren.

Die ersten Messdaten und die zweiten Messdaten werden zu einer überlagerten Aufnahme der Zahnsituation zusammengefügt.

Anhand der zweiten Messdaten der festgelegten Objektbereiche werden die Lage und die Orientierung der Implantate und/oder der Präparationen relativ zueinander und relativ zu den Zähnen ermittelt.

Das Verfahren zur Vermessung der Zahnsituation kann sowohl auf eine Zahnsituation in der Mundhöhle des Patienten als auch auf ein Zahnmodell der Zahnsituation angewendet werden. Das Zahnmodell kann beispielsweise durch einen Gipsabdruck der Zahnsituation hergestellt sein. Die Implantate können beliebig gestaltet sein und eine bestimmte Anschlussgeometrie für ein einzusetzendes Abutment oder eine passende Zahnrestauration aufweisen. Zur Verbesserung der Vermessung können Messkörper verwendet werden, die mit den Implantaten verbunden werden. Die Messkörper können bestimmte Messgeometrien aufweisen, die eine Bestimmung der Lage und Orientierung der Implantate ermöglicht. Dabei kann die Messgeometrie eine bestimmte geometrische Form, wie ein Vieleck, oder beispielsweise drei in einem Dreieck angeordnete Punkte aufweisen. Die Messkörper können dabei so ausgestaltet sein, dass sie zur Vermessung mittels des ersten Messverfahrens und des zweiten Messverfahrens geeignet sind. Bei der Vermessung mittels eines Röntgenaufnahmeverfahrens können die Messkörper beispielsweise röntgensensitiv sein. Das vorliegende Verfahren kann auch zur Vermessung von Präparationen verwendet werden. Der erste Bereich kann einen Teil der Zahnsituation mit mindestens zwei Implantaten oder auch die gesamte Zahnsituation umfassen. Die erzeugten ersten Messdaten können dreidimensionale Bilddaten oder auch Rohdaten zur späteren Verarbeitung sein. Die Objektbereiche können beliebig geformt sein, beispielsweise können sie eine Kreisform um die Implantate aufweisen. Die zweiten Messdaten können dreidimensionale Bilddaten oder Rohdaten zur weiteren Verarbeitung sein. Die zweiten Messdaten sind präziser beispielsweise bezüglich der Auflösung und Genauigkeit im Vergleich zu den ersten Messdaten. Die Abweichung der zweiten Messdaten von den tatsächlichen Abmessungen des aufgenommenen Objekts ist also geringer als bei den ersten Messdaten.

Das Zusammenfügen der ersten Messdaten und der zweiten Messdaten zu der überlagerten Aufnahme kann mittels eines Mustererkennungsalgorithmus erfolgen, der übereinstimmende Bereiche erkennt. Die überlagerte Aufnahme ermöglicht dem Benutzer einen schnellen Überblick über die gesamte Zahnsituation mit den integrierten präziseren zweiten Messdaten der Objektbereiche um die Implantate.

Zur Verbesserung der Vermessung können Messkörper auf die Implantate aufgesetzt werden, die eine Bestimmung der Lage und der Orientierung der Implantate erleichtern. Die Messkörper oder die sichtbaren Bereiche der eingesetzten Implantate können beispielsweise mittels eines Computeralgorithmus in den zweiten Messdaten erkannt werden, wobei anschließend die Lage und die Orientierung der Implantate relativ zueinander und relativ zu den Zähnen ebenfalls mittels eines Computeralgorithmus automatisch ermittelt werden können.

Ein Vorteil dieses Verfahrens liegt darin, dass zunächst eine Übersichtsaufnahme mittels des ersten Messverfahrens und anschließend eine präzisere Aufnahme mittels des zweiten Messverfahrens erzeugt werden. Dies ermöglicht eine genauere Vermessung der Implantate zur Bestimmung der Lage und Orientierung der Implantate relativ zueinander und relativ zur Zahnsituation, wobei die Vermessungsdauer verkürzt wird.

Vorteilhafterweise können die Objektbereiche manuell durch einen Benutzer festgelegt werden.

Bei der manuellen Festlegung der Objektbereiche kann der Benutzer mittels eines Computers die Objektbereiche mit einem Mauszeiger umranden. Der Benutzer kann auch ein virtuelles Werkzeug benutzen, wobei zunächst ein Mittelpunkt eines kreisförmigen Objektbereichs an eine Symmetrieachse eines Implantats festgelegt wird und anschließend ein Kreis um diesen Punkt mit einem passenden Abstand gezogen wird.

Vorteilhafterweise können die Objektbereiche automatisch mittels eines Suchalgorithmus festgelegt werden.

Bei der automatischen Festlegung der Objektbereiche können die Implantate mittels des Suchalgorithmus computergestützt erkannt werden. Anschließend kann automatisch ein kreisförmiger Objektbereich um jeden der erkannten Implantate mit einem bestimmten Abstand zur Symmetrieachse des Implantats festgelegt werden. Der Abstand zum Mittelpunkt kann beispielsweise 2 mm bis 10 mm betragen.

Vorteilhafterweise kann das erste Messverfahren auf einem Streifenprojektionsverfahren, auf einem konfokalen Mikroskopieverfahren, auf einem Weißlichtinterferometrieverfahren, auf einem Triangulationsverfahren mit farblichen Mustern oder auf einem dreidimensionalen Röntgenaufnahmeverfahren beruhen.

Bei dem bekannten Streifenprojektionsverfahren wird das Messobjekt mit einem Streifenmuster aus parallelen hellen und dunklen Streifen unterschiedlicher Breite beleuchtet. In einem weiteren Schritt wird das projizierte Streifenmuster unter einem bekannten Blickwinkel zur Projektion mittels einer Kamera aufgenommen. Unter Verwendung eines sogenannten Phasenschiebeverfahrens kann eine Projektionskoordinate bestimmt werden, die die Nummer des Streifens wiedergibt. Die Nummer des Streifens im Projektor entspricht einer Bildkoordinate in der Kamera. Bei einer bekannten Kameraposition und einer bekannten Projektorposition relativ zum Objekt kann ein Schnittpunkt zwischen einer Ebene, die durch den jeweiligen Streifen definiert ist und einer Geraden, die durch die Koordinate in der Kamera definiert ist, berechnet werden. Für jeden der Messpunkte wird auf diese Weise die dreidimensionale Koordinate der Oberfläche bestimmt.

Bei der Weißlichtinterferometrie wird ein Licht geringer Kohärenzlänge verwendet, sodass farbige Indifferenzen entstehen, wenn die Weglängen im Referenz- und Objektstrahl nahezu gleich sind. Beim Verändern der Weglänge wird das Interferenzmuster verändert, sodass anhand des Interferenzmusters der Abstand zur Oberfläche des Messobjekts bestimmt werden kann.

Bei dem dreidimensionalen konfokalen Mikroskopieverfahren wird die Oberfläche des dentalen Objekts schrittweise abgetastet, wobei eine Fokalebene schrittweise verschoben wird. Das Licht außerhalb der Fokalebene wird mittels einer Lochblende möglichst ausgeblendet. Aus den gemessenen Bilddaten der einzelnen Schritte unterschiedlicher Fokalebenen kann anschließend ein dreidimensionales Modell des vermessenen Objekts berechnet werden.

Bei dem Triangulationsverfahren mit farblichen Mustern können mehrere Lichtquellen unterschiedlicher Farbe oder eine Lichtquelle mit mehreren Filtern unterschiedlicher Farbe und ein Projektionsgitter zur Erzeugung der projizierten farblichen Muster verwendet werden. Dadurch können klar von einander abgegrenzte farbliche Muster, wie parallele Linien unterschiedlicher Farbe, erzeugt werden, die auf das dentale Objekt projiziert werden. Dieses Verfahren kann als das erste Messverfahren zur Erzeugung der ersten Messdaten und/oder als das zweite Messverfahren zur Erzeugung der zweiten Messdaten verwendet werden.

Das dreidimensionale Röntgenaufnahmeverfahren kann beispielsweise ein DVT- oder CT-Verfahren sein. Das erste Messverfahren oder das zweite Messverfahren kann auch ein MRT-Verfahren sein.

Vorteilhafterweise kann das zweite, präzisere Messverfahren auf einem Triangulationsverfahren und auf einem Streifenprojektionsverfahren beruhen, wobei zuvor zumindest an den aufzunehmenden Objektbereichen eine Puderung erfolgt.

Für eine präzise Aufnahme ist eine nicht spiegelnde Oberfläche des vermessenen Objekts zwingend erforderlich. Dafür wird das dentale Objekt meist vor der Aufnahme mit einem speziellen Puder beschichtet. Nach der Aufnahme wird die aufgetragene Puderschicht entfernt. Bei einer fehlenden Puderung wird lediglich eine geringe Genauigkeit erreicht, da Aufnahmefehler durch ungleichmäßige Reflexionen erzeugt werden.

Vorteilhafterweise kann das zweite, präzisere Messverfahren auf einem Triangulationsverfahren beruhen, bei dem ein zweiter Triangulationswinkel geringer als ein erster Triangulationswinkel des ersten Messverfahrens sein kann und so klein gewählt ist, dass die Genauigkeitsanforderungen an die zweiten Messdaten erfüllt sind.

Der zweite Triangulationswinkel des zweiten präziseren Messverfahrens ist so klein gewählt, dass die Genauigkeitsanforderungen, wie beispielsweise eine ausreichende Auflösung, erfüllt sind.

Vorteilhafterweise kann das zweite, präzisere Messverfahren mittels eines Multikamera-Systems durchgeführt werden und auf einem Photogrammetrieverfahren beruhen.

Das Photogrammetrieverfahren ist eine Messmethode und Auswerteverfahren der Fernerkundung, um aus Aufnahmen und genauen Messbildern eines Objektes aus unterschiedlichen Raumrichtungen seine räumliche Lage oder dreidimensionale Form zu bestimmen. Im Regelfall werden die Bilder mit einem speziellen Multikamera-System aufgenommen. Mittels dieses Verfahrens kann aus den zweidimensionalen optischen Aufnahmen der einzelnen Kameras des Multikamera-Systems eine dreidimensionale Aufnahme des aufzunehmenden Objekts berechnet werden.

Vorteilhafterweise kann das zweite, präzisere Messverfahren mittels eines taktilen Tastscanners durch eine punktweise Abtastung der Objektbereiche durchgeführt werden.

Der taktile Tastscanner kann eine Vorrichtung sein, die die Objektbereiche Punkt für Punkt abtastet und für jeden Objektpunkt eine Tiefenkoordinate erzeugt wird. Aus den ermittelten Tiefenkoordinaten kann dann eine dreidimensionale Oberfläche des zu vermessenden Objekts erzeugt werden.

Vorteilhafterweise kann das zweite, präzisere Messverfahren auf einem dreidimensionalen Röntgenaufnahmeverfahren mit einer höheren Auflösung als beim ersten Messverfahren beruhen.

Bei einem DVT- oder CT-Verfahren kann die Auflösung des erzeugten dreidimensionalen Röntgenbildes durch eine Verminderung der Schnittfolge erzielt werden, die mit einer erhöhten Dosisbelastung verbunden ist.

Vorteilhafterweise kann das zweite, präzisere Messverfahren mittels eines Multikamera-Systems durchgeführt werden und auf einem Photogrammetrieverfahren beruhen, wobei die Objektbereiche in jeder der einzelnen Aufnahmen des Multikamera-Systems festgelegt werden können. Anschließend kann anhand der zweiten Messdaten der Objektbereiche aus den einzelnen Aufnahmen eine dreidimensionale Aufnahme der Objektbereiche mittels eines Computeralgorithmus rekonstruiert werden.

Dadurch werden lediglich die Objektbereiche zur Rekonstruktion der dreidimensionalen Aufnahme verwendet. Dies führt zu einer kürzeren Rechenzeit bei der Rekonstruktion der dreidimensionalen Aufnahme.

### Kurzbeschreibung der Zeichnungen

Die Erfindung wird anhand der Zeichnungen erläutert. Es zeigt, die
- Fig. 1: eine Skizze der Zahnsituation zur Verdeutlichung des Verfahrens;
- Fig. 2: eine Skizze zur Verdeutlichung der automatischen Festlegung eines Objektbereichs.

### Ausführungsbeispiel

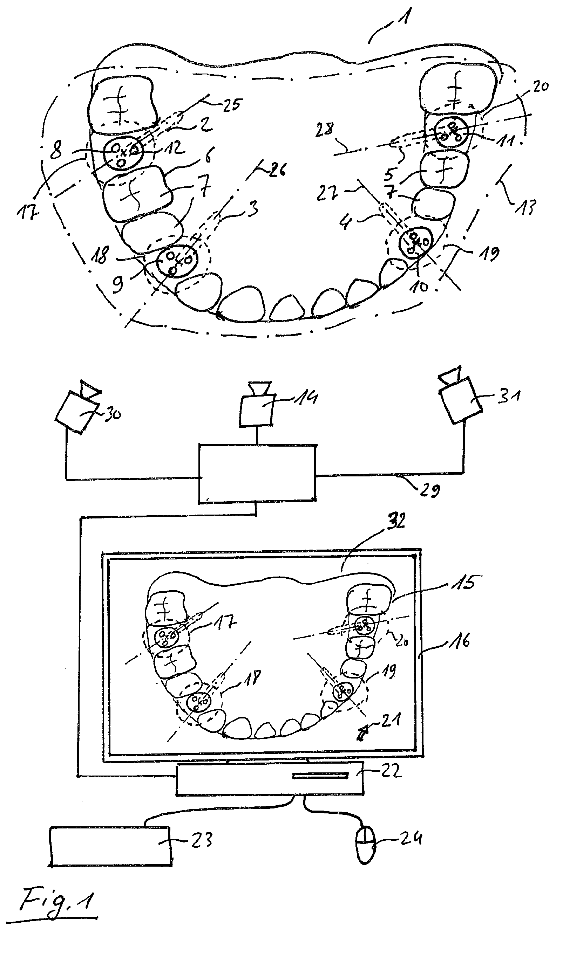

Die Fig. 1 zeigt eine Skizze zur Verdeutlichung des Verfahrens zur Vermessung einer Zahnsituation 1 umfassende mehrere Implantate 2, 3, 4 und 5 zum Einsetzen von Zahnrestaurationen. Ein Restzahnbestand 6 umfasst mehrere benachbarte Zähne 7. Das erste Implantat 2 ist mit einem ersten Messkörper 8, das zweite Implantat 3 ist mit einem zweiten Messkörper 9, das dritte Implantat 4 ist mit einem dritten Messkörper 10 und das vierte Implantat 5 ist mit einem vierten Messkörper 11 verbunden. Die Messkörper 8, 9, 10 und 11 weisen eine Messgeometrie 12, bestehend aus drei Punkten, auf, die die Ermittlung der Lage und der Orientierung der Implantate 2, 3, 4 und 5 relativ zu den Zähnen 7 und relativ zueinander ermöglicht: Im ersten Verfahrensschritt wird ein erster Bereich 13, der durch eine Strichpunktlinie dargestellt ist, mittels einer ersten Kamera 14 unter Verwendung eines ersten Messverfahrens vermessen. Dabei wird eine Übersichtsaufnahme der gesamten Zahnsituation 1 erzeugt. Das erste Messverfahren kann beispielsweise auf einem Streifenprojektionsverfahren, auf einem konfokalen Mikroskopieverfahren, auf einem Weißlichtinterferometrieverfahren, auf einem Triangulationsverfahren mit farblichen Mustern oder auf einem dreidimensionalen Röntgenaufnahmeverfahren beruhen. Die Übersichtsaufnahme 15 wird mittels einer Anzeigevorrichtung 16, wie einem Monitor, angezeigt. Anschließend werden in den nächsten Verfahrensschritten die Objektbereiche 17, 18, 19 und 20 um die Implantate 2, 3, 4 und 5 festgelegt. Die Objektbereiche können entweder manuell durch den Benutzer mittels eines Mauszeigers 21 oder automatisch festgelegt werden. Zur weiteren Bildbearbeitung wird ein Computer 22 mit Eingabemitteln, wie einer Tastatur 23 und einer Maus 24, verwendet. Die Objektbereiche 17, 18, 19 und 20 sind durch eine gestrichelte Linie dargestellt und sind kreisförmig mit einem Mittelpunkt ausgebildet, der mit einer Symmetrieachse 25, 26, 27 und 28 der Implantate 2, 3, 4 und 5 übereinstimmt. Anschließend werden die festgelegten Objektbereiche 17, 18, 19 und 20 mittels eines Multikamera-Systems 29, bestehend aus einer ersten Kamera 30 und einer zweiten Kamera 31 erfasst. Dabei werden zweite Messdaten erzeugt die präziser sind als die ersten Messdaten der Übersichtsaufnahme 15. Die zweiten Messdaten weisen eine höhere Auflösung auf als die ersten Messdaten und erlauben damit eine genauere Bestimmung der Lage und der Orientierung der Implantate 2, 3, 4 und 5. Anschließend werden die ersten Messdaten der Übersichtsaufnahme 15 und die zweiten Messdaten der festgelegten Objektbereiche zu einer überlagerten Aufnahme 32 zusammengefügt, die mittels der Anzeigevorrichtung 16 dargestellt wird. Dadurch kann der Benutzer sich schnell orientieren und anhand der Objektbereiche 17, 18, 19 und 20 die Lage und Orientierung der Implantate 2, 3, 4 und 5 bestimmen. Die Bestimmung der Lage und Orientierung der Implantate 2, 3, 4 und 5 kann auch automatisch mittels eine Computeralgorithmus erfolgen, wobei die Messkörper 8, 9, 10 und 11 anvisiert werden. Das Ergebnis des vorliegenden Verfahrens ist also die genaue Lage und Orientierung der Implantate 2, 3, 4 und 5 zueinander und relativ zu den Zähnen 7. Das in Fig. 1 dargestellte Multikamera-System 29 ist zur Durchführung eines Photogrammetrieverfahrens geeignet, wobei die Objektbereiche 17, 18, 19 und 20 in jeder der einzelnen Aufnahmen der Kameras 30 und 31 festgelegt werden und anschließend aus den zweiten Messdaten dieser festgelegten Objektbereiche eine dreidimensionale Aufnahme der Objektbereiche 17, 18, 19 und 20 rekonstruiert wird.

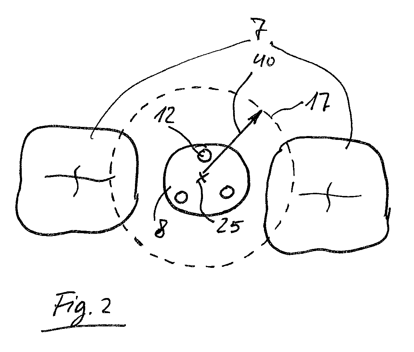

Die Fig. 2 zeigt eine Skizze zur Verdeutlichung einer automatischen Festlegung des Objektbereichs 17 zwischen den Nachbarzähnen 7, wobei der Rand des Objektbereichs 17 automatisch als ein Kreis mit einem bestimmten Abstand 40 und die Symmetrieachse 25 des Implantats 2 festgelegt wird. Der Abstand kann beispielsweise zwischen 4 mm und 10 mm betragen. Die Lage der Symmetrieachse 25 kann mittels der Messgeometrie 12 des Messkörpers 8 automatisch bestimmt werden.

### Bezugszeichen

- 1: Zahnsituation
- 2: Implantat
- 3: Implantat
- 4: Implantat
- 5: Implantat
- 6: Restzahnbestand
- 7: Zähne
- 8: Messkörper
- 9: Messkörper
- 10: Messkörper
- 11: Messkörper
- 12: Messgeometrie
- 13: Bereich
- 14: Kamera
- 15: Übersichtsaufnahme
- 16: Anzeigevorrichtung
- 17: Objektbereich
- 18: Objektbereich
- 19: Objektbereich
- 20: Objektbereich
- 21: Mauszeiger
- 22: Computer
- 23: Tastatur
- 24: Maus
- 25: Symmetrieachse
- 26: Symmetrieachse
- 27: Symmetrieachse
- 28: Symmetrieachse
- 29: Multikamera-System
- 30: erste Kamera
- 31: zweite Kamera
- 32: gesamte Aufnahme
- 40: Abstand/ Radius

## Patentansprüche

1. Verfahren zur Vermessung einer Zahnsituation (1) umfassend mehrere Implantate (2, 3, 4, 5) und/oder Präparationen zum Einsetzen von Zahnrestaurationen, wobei unter Verwendung eines ersten Messverfahrens ein erster Bereich (13) der Zahnsituation (1) erfasst wird und dabei erste Messdaten erzeugt werden, wobei der erste Bereich (13) mindestens zwei Implantate (2, 3, 4, 5) und/oder Präparationen umfasst, wobei Objektbereiche (17, 18, 19, 20) um die Implantate (2, 3, 4, 5) und/oder die Präparationen festgelegt werden, wobei unter Verwendung eines zweiten Messverfahrens die festgelegten Objektbereiche (17, 18, 19, 20) erfasst werden und dabei zweite Messdaten erzeugt werden, wobei das zweite Messverfahren präziser ist als das erste Messverfahren, wobei die ersten Messdaten und die zweiten Messdaten zu einer überlagerten Aufnahme (32) der Zahnsituation (1) zusammengefügt werden, **dadurch gekennzeichnet, dass** anhand der zweiten Messdaten der festgelegten Objektbereiche (17, 18, 19, 20) die Lage und die Orientierung der Implantate (2, 3, 4, 5) und/oder der Präparationen relativ zueinander und relativ zu den Zähnen (7) ermittelt werden.

2. Verfahren nach Anspruch 1, **dadurch gekennzeichnet, dass** die Objektbereiche (17, 18, 19, 20) manuell durch einen Benutzer festgelegt werden.

3. Verfahren nach Anspruch 1, **dadurch gekennzeichnet, dass** die Objektbereiche (17, 18, 19, 20) automatisch mittels eines Suchalgorithmus festgelegt werden.

4. Verfahren nach einem der Ansprüche 1 bis 3, **dadurch gekennzeichnet, dass** das erste Messverfahren auf einem Streifenprojektionsverfahren, auf einem konfokalen Mikroskopieverfahren, auf einem Weißlichtinterferometrieverfahren, auf einem Triangulationsverfahren mit farblichen Mustern oder auf einem dreidimensionalen Röntgenaufnahmeverfahren beruht.

5. Verfahren nach einem der Ansprüche 1 bis 4, **dadurch gekennzeichnet, dass** das zweite, präzisere Messverfahren auf einem Triangulationsverfahren und auf einem Streifenprojektionsverfahren beruht, wobei zuvor zumindest an den aufzunehmenden Objektbereichen (17, 18, 19, 20) eine Puderung erfolgt.

6. Verfahren nach Anspruch 4 oder 5, **dadurch gekennzeichnet, dass** das zweite, präzisere Messverfahren auf einem Triangulationsverfahren beruht, bei dem ein zweiter Triangulationswinkel geringer als ein erster Triangulationswinkel des ersten Messverfahrens ist und so klein gewählt ist, dass die Genauigkeitsanforderungen an die zweiten Messdaten erfüllt sind.

7. Verfahren nach einem der Ansprüche 1 bis 4, **dadurch gekennzeichnet, dass** das zweite, präzisere Messverfahren mittels eines Multikamera-Systems (29) durchgeführt wird und auf einem Photogrammetrieverfahren beruht.

8. Verfahren nach einem der Ansprüche 1 bis 4, **dadurch gekennzeichnet, dass** das zweite, präzisere Messverfahren mittels eines taktilen Tastscanners durch eine punktweise Abtastung der Objektbereiche durchgeführt wird.

9. Verfahren nach einem der Ansprüche 1 bis 4, **dadurch gekennzeichnet, dass** das zweite, präzisere Messverfahren auf einem dreidimensionalen Röntgenaufnahmeverfahren mit einer höheren Auflösung als beim ersten Messverfahren beruht.

10. Verfahren nach einem der Ansprüche 1 bis 6, **dadurch gekennzeichnet, dass** das zweite, präzisere Messverfahren mittels eines Multikamera-Systems (29) durchgeführt wird und auf einem Photogrammetrieverfahren beruht, wobei die Objektbereiche (17, 18, 19, 20) in jeder der einzelnen Aufnahmen des Multikamera-Systems (29) festgelegt werden und anschließend anhand der zweiten Messdaten der Objektbereiche (17, 18, 19, 20) aus den einzelnen Aufnahmen eine dreidimensionale Aufnahme der Objektbereiche (17, 18, 19, 20) mittels eines Computeralgorithmus rekonstruiert wird.

## Claims

1. A method for measuring a dental condition (1) comprising several implants (2, 3, 4, 5) and/or preparations for attaching dental restorations, wherein, using a first measuring method, a first region (13) of the dental condition (1) is detected and therewith first measured data generated, wherein the first region (13) comprises at least two implants (2, 3, 4, 5) and/or preparations, wherein object areas (17, 18, 19, 20) around the implants (2, 3, 4, 5) and/or the preparations are determined, wherein, using a second measuring method, the determined object areas (17, 18, 29, 20) are detected and therewith second measured data generated, wherein the second measuring method is more precise than the first measuring method, wherein the first measured data and the second measured data are combined into one overlaid image (32) of the dental condition (1), **characterized in that** the position and orientation of the implants (2, 3, 4, 5) and/or the preparations relative to each other and relative to the teeth (7) are determined based upon the second measured data of the determined object areas (17, 18, 19, 20).

2. The method according to claim 1, **characterized in that** the object areas (17, 18, 19, 20) are determined by a user manually.

3. The method according to claim 1, **characterized in that** the object areas (17, 18, 19, 20) are determined automatically by means of a search algorithm.

4. The method according to one of claims 1 through 3, **characterized in that** the first measuring method is based upon a fringe projection method, upon a confocal microscopy method, upon a white light interferometry method, upon a triangulation method with colored patterns, or upon a three-dimensional radiography method.

5. The method according to one of claims 1 through 4, **characterized in that** the second, more precise measuring method is based upon a triangulation method and upon a fringe projection method, wherein a powdering of at least the object areas (17, 18, 19, 20) to be acquired takes place beforehand.

6. The method according to claim 4 or 5, **characterized in that** the second, more precise measuring method is based upon a triangulation method, in which a second triangulation angle is less than a first triangulation angle of the first measuring method and is selected to be so small that the precision requirements for the second measured data are met.

7. The method according to one of claims 1 through 4, **characterized in that** the second, more precise measuring method is carried out by means of a multi-camera system (29) and is based upon a photogrammetry method.

8. The method according to one of claims 1 through 4, **characterized in that** the second, more precise measuring method is carried out by means of a tactile scanner by scanning the object areas point by point.

9. The method according to one of claims 1 through 4, **characterized in that** the second, more precise measuring method is based upon a three-dimensional radiography method with a higher resolution than in the first measuring method.

10. The method according to one of claims 1 through 6, **characterized in that** the second, more precise measuring method is carried out by means of a multi-camera system (29) and is based upon a photogrammetry method, wherein the object areas (17, 18, 19, 20) are determined in each of the individual images of the multi-camera system (29) and, subsequently, based upon the second measured data of the object areas (17, 18, 19, 20) from the individual images, a three-dimensional image of the object areas (17, 18, 19, 20) is reconstructed using a computer algorithm.

## Revendications

1. Procédé de mesurage d'une situation dentaire (1) comprenant plusieurs implants (2, 3, 4, 5) et/ou préparations pour l'insertion de restaurations dentaires, dans lequel, par le recours à une premier procédé de mesurage, une première zone (13) de la situation dentaire (1) est capturée et ainsi des premières données de mesurage sont produites, dans lequel la première zone (13) comprend au moins deux implants (2, 3, 4, 5) et/ou préparations, dans lequel des zones d'objet (17, 18, 19, 20) sont définies autour des implants (2, 3, 4, 5) et/ou préparations, dans lequel par le recours à un second procédé de mesurage, les zones d'objet définies (17, 18, 19, 20) sont capturées et ainsi des deuxièmes données de mesurage sont produites, dans lequel le deuxième procédé de mesurage est plus précis que le premier procédé de mesurage, dans lequel les premières données de mesurage et les deuxièmes données de mesurage sont fusionnées en un enregistrement superposé (32) de la situation dentaire (1), **caractérise en ce que** grâce aux deuxièmes données de mesurage des zones d'objet définies (17, 18, 19, 20), on détermine l'emplacement et l'orientation des implants (2, 3, 4, 5) et/ou des préparations les uns par rapport aux autres et par rapport aux dents (7).

2. Procédé selon la revendication 1, **caractérisé en ce que** les zones d'objet (17, 18, 19, 20) sont définies manuellement par un utilisateur.

3. Procédé selon la revendication 1, **caractérisé en ce que** les zones d'objet (17, 18, 19, 20) sont définies automatiquement par un algorithme de recherche.

4. Procédé selon l'une des revendications 1 à 3, **caractérisé en ce que** le premier procédé de mesurage est basé sur une technique de projection de franges, sur une méthode de microscopie à foyer commun, sur une méthode d'interféromètres par lumière blanche, sur une méthode de triangulation avec des échantillons de couleur ou sur un procédé de radiographie tridimensionnelle.

5. Procédé selon l'une des revendications 1 à 4, **caractérisé en ce que** le deuxième procédé de mesurage, plus précis, est basé sur une méthode de triangulation et sur une technique de projection de franges, dans laquelle un poudrage est effectué au préalable, au moins dans les zones d'objet à enregistrer (17, 18, 19, 20).

6. Procédé selon la revendication 4 ou 5, **caractérisé en ce que** le deuxième procédé de mesurage, plus précis, est basé sur une méthode de triangulation dans lequel un deuxième angle de triangulation est plus petit qu'un premier angle de triangulation du premier procédé de mesurage et il est choisi pour être suffisamment petit pour que les exigences de précision imposées aux deuxièmes données de mesurage soient respectées.

7. Procédé selon l'une des revendications 1 à 4, **caractérisé en ce que** le deuxième procédé de mesurage, plus précis, est mis en oeuvre par le biais d'un système multi-caméras (29) et qu'il fait appel à un procédé de photogrammétrie.

8. Procédé selon l'une des revendications 1 à 4, **caractérisé en ce que** le deuxième procédé de mesurage, plus précis, est mis en oeuvre par le biais d'un scanner palpeur tactile effectuant un balayage ponctuel des zones d'objet.

9. Procédé selon l'une des revendications 1 à 4, **caractérisé en ce que** le deuxième procédé de mesurage, plus précis, est basé sur un procédé de radiographie tridimensionnel dont la résolution est plus haute que dans le premier procédé de mesurage.

10. Procédé selon l'une des revendications 1 à 6, **caractérisé en ce que** le deuxième procédé de mesurage, plus précis, est effectué par le biais d'un système multi-caméras (29) et qui est basé sur un procédé de photogrammétrie, dans lequel les zones d'objet (17, 18, 19, 20) sont définies dans chacun des enregistrements individuels du système multi-caméras (29) et dans lequel un enregistrement tridimensionnel des zones d'objet (17, 18, 19, 20) est ensuite reconstruit d'après les deuxièmes données de mesurage des zones d'objet (17, 18, 19, 20) à partir des enregistrements individuels par le biais d'un algorithme informatique.
